# EUROPEAN PATENT SPECIFICATION

(11) **EP 0 563 238 B1**
(45) Date of publication and mention of the grant of the patent: **22.05.2002**
(21) Application number: 92902616.9
(22) Date of filing: 13.12.1991
(51) Int. Cl.: A61K 31/415, C07D 409/06

(54) **IMIDAZOLYL-ALKENOIC ACIDS**
IMIDAZOLYL-ALKENCARBONSÄUREN
ACIDES IMIDAZOLYL-ALCENOIQUES

(30) Priority: 14.12.1990 US 627177; 14.12.1990 US 629123
(43) Date of publication of application: 06.10.1993
(73) Proprietor: SMITHKLINE BEECHAM CORPORATION, Philadelphia Pennsylvania 19101 (US)
(72) Inventor: FINKELSTEIN, Joseph Alan, Philadelphia, PA 19151 (US); KEENAN, Richard McCulloch, Malvern, PA 19355 (US); WEINSTOCK, Joseph, Phoenixville, PA 19460 (US)
(74) Representative: Thompson, Clive Beresford
(86) International application number: US9109340
(87) International publication number: WO9210189

(56) References cited:
- EP-A- 0 028 833
- EP-A- 0 253 310
- EP-A- 0 291 969
- EP-A- 0 403 158
- EP-A- 0 403 159
- US-A- 4 340 598
- US-A- 5 185 351

## Description

The present invention relates to new imidazolyl-alkenoic acids which are angiotensin II receptor antagonists and are useful in regulating hypertension induced or exacerbated by angiotensin II, and in the treatment of congestive heart failure, renal failure, and glaucoma. This invention also relates to pharmaceutical compositions containing these compounds and methods for using these compounds as antagonists of angiotensin II, as antihypertensive agents and as agents for treating congestive heart failure, renal failure, and glaucoma.

### BACKGROUND OF THE INVENTION

The class of peptide pressor hormone known as angiotensin is responsible for a vasopressor action that is implicated in the etiology of hypertension in man. Inappropriate activity of the renin-angiotensin systems appears to be a key element in essential hypertension, congestive heart failure and in some forms of renal disease. In addition to a direct action on arteries and arterioles, angiotensin II (AII), being one of the most potent endogenous vasoconstrictors known, exerts stimulation on the release of aldosterone from the adrenal cortex. Therefore, the renin-angiotensin system, by virtue of its participation in the control of renal sodium handling, plays an important role in cardiovascular hemeostasis.

Interruption of the renin-angiotensin system with converting enzyme inhibitors, such as captopril, has proved to be clinically useful in the treatment of hypertension and congestive heart failure (Abrams, W.B., et al., (1984), Federation Proc., 43, 1314). The most direct approach towards inhibition of the renin-angiotensin system would block the action of AII at the receptor. Compelling evidence suggests that All also contributes to renal vasoconstriction and sodium retention that is characteristic of a number of disorders such as heart failure, cirrhosis and complications of pregnancy (Hollenberg, N.K., (1984), J. Cardiovas. Pharmacol., 6, S176). In addition, recent animal studies suggest that inhibition of the renin-angiotensin system may be beneficial in halting or slowing the progression of chronic renal failure (Anderson, S., et al., (1985), J. Clin. Invest., 76, 612). Also, a recent patent application (South African Patent Application No. 87/01,653) claims that AII antagonists are useful as agents for reducing and controlling elevated intraocular pressure, especially glaucoma, in mammals.

The compounds of this invention inhibit, block and antagonize the action of the hormone AII, and are therefore useful in regulating and moderating angiotensin induced hypertension, congestive heart failure, renal failure and other disorders attributed to the actions of AII. When compounds of this invention are administered to mammals, the elevated blood pressure due to All is reduced and other manifestations based on AII intercession are minimized and controlled. Compounds of this invention are also expected to exhibit diuretic activity.

Recognition of the importance of blocking and inhibiting the actions of All has stimulated other efforts to synthesize antagonists of AII. The following references have disclosed imidazole derivatives which are described as having AII blocking activity and useful as hypotensive agents.

Furukawa et al., U.S. Patent 4,340,598 discloses imidazol-5-yl-acetic acids and imidazol-5-yl-propanoic acids. Specifically, the discloser includes 1-benzyl-2-n-butyl-5-chloroimidazole-4-acetic acid and 1-benzyl-2-phenyl-5-chloroimidazole-4-propanoic acid.

Furukawa, et al., U.S. Patent 4,355,040 discloses substituted imidazole-5-acetic acid derivatives. A compound specifically disclosed is 1-(2-chlorobenzyl)-2-n-butyl-4-chloroimidazole-5-acetic acid.

Carini et al. in EP 253,310 disclose certain imidazolylpropenoic acids. Two intermediates described in this patent are ethyl 3-[1-(4-nitrobenzyl)-2-butyl-4-chloroimidazol-5-yl]propenoate and ethyl 3-[2-butyl-4-chloro-1-(4-aminobenzyl)imidazol-5-yl]propenoate.

Also, Wareing, in PCT/EP 86/00297, discloses as intermediates certain imidazolylpropenoate compounds. On page 62, Formula (CX) is ethyl 3-[1(-4-fluorophenyl)-4-isopropyl-2-phenyl-1H-imidazol-5-yl]-2-propenoate.

### DESCRIPTION OF THE INVENTION

The compounds of the present invention that are blockers of angiotensin II receptors are represented by the following Formula (I): 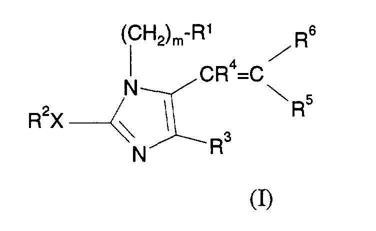 in which:
R¹ is phenyl unsubstituted or substituted by one to three substituents selected from chloro, fluoro, trifluoromethyl, nitro, methyl, methoxy, hydroxy, sulfonamido, cyano, carboxy, carboC₁-C₆alkoxy, carbamoyl, or tetrazol-5-yl;
m is 0 or 2;
X is a single bond or S;
R² is C₂-C₈alkyl;
R³ is hydrogen, chloro, fluoro, or trifluoromethyl;
R⁴ is hydrogen or C₁-C₄alkyl;
R⁵ is thienylmethyl, furylmethyl, imidazolylmethyl, or pyridylmethyl, each of which is unsubstituted or substituted by methyl or methoxy; or R⁵ is benzyl unsubstituted or substituted by one to three substituents selected from Cl, Br, F, NO₂, OCH₃, OH, CF₃, NR⁷R⁷, CH₃, or CO₂R⁷, or by methylenedioxy;
R⁶ is CO₂H, CO₂C₁-C₂alkyl, or CONH₂; and
each R⁷ independently is hydrogen, C₁-C₆alkyl, or (CH₂)_{q} phenyl, wherein q is 0-4;
or a pharmaceutically acceptable salt thereof.

Preferred compounds of this invention are represented by Formula (I) when each R⁷ independently is H or CH₃.

The E isomers (trans stereochemistry of the carboxy and imidazole groups) are generally more active and thus, are preferred over the Z isomers (cis).

As used herein, the term alkyl mean a carbon chains which is branched or unbranched with the length of the chain determined by the descriptor preceding the term.

Particular compounds of the invention include, but are not limited to, the following:
(E)-3-[2-n-butyl-1-{4-carboxyphenyl}-1H-imidazo-5-yl]-2-(2-thienyl)methyl-2-propenoic acid and
(E)-3-[2-n-butyl-1-{(4-carboxyphenyl)ethyl}-1H-imidazo-5-yl]-2-(2-thienyl)methyl-2-propenoic acid;
   or a pharmaceutically acceptable salt thereof.

The invention also relates to pharmaceutical compositions comprising a pharmaceutical carrier and an effective amount of a compound of Formula (I).

Also included in the present invention are methods for antagonizing angiotensin II receptors which comprises administering to a subject in need thereof an effective amount of a compound of Formula (I). Methods of producing antihypertensive activity and methods of treating congestive heart failure, glaucoma, and renal failure by administering these compounds are also included in this invention.

The compounds of this invention are prepared by procedures described herein and illustrated by the examples. Reagents, protecting groups and functionality on the imidazole and other fragments of the molecule must be consistent with the proposed chemical transformations. Steps in the synthesis must be compatible with the functional groups and the protecting groups on the imidazole and other parts of the molecule.

The starting materials, 2-R²X-imidazole, are known to the art (J. Org. Chem. 45:4038, 1980) or are synthesized by known procedures. For example, imidazole is converted to 2-n-butylimidazole by reacting imidazole with triethylorthoformate and p-toluenesulfonic acid to give 1-diethoxyorthoamide imidazole and then treating with n-butyl lithium to give the 2-lithium derivative of the orthoamide and alkylating with n-butyl iodide in a suitable solvent, such as tetrahydrofuran (THF).

The following procedure is useful for the preparation of compounds of Formula (I) particularly where R¹ is 2-chlorophenyl or 4-carboxyphenyl, R² is n-butyl or n-propyl, X is a single bond or S, R³ is hydrogen, chloro, or CF₃, R⁴ is hydrogen, R⁵ is as described in Formula (I) and R⁶ is COOH or CO₂C₁-C₂alkyl.

The 1-R¹(CH₂)ₘ-group is incorporated onto the 2-R²X-imidazole by known procedures, for example, by reaction with an R¹-(CH₂)ₘ halide, mesylate or acetate, such as 2-chlorobenzyl bromide, in a suitable solvent, such as dimethylformamide (DMF), in the presence of a suitable acid acceptor, such as sodium alkylate, potassium or sodium carbonate, or a metal hydride, preferably sodium hydride at a reaction temperature of 25°C to 100°C, preferably 50°C. The resulting 1-R¹(CH₂)ₘ-2-R²X imidazole is hydroxymethylated in the 5-position, for example, by reacting with formaldehyde in the presence of sodium acetate in acetic acid to provide the 1-R¹(CH₂)ₘ-2-R²X-5-hydroxymethyl-imidazole intermediates.

Alternatively, the 1-R¹(CH₂)ₘ-2-R²-5-hydroxymethyl-imidazole intermediates are prepared by reacting an imido ether, R²-C(=NH)-O-alkyl, such as valeramidine methyl ether, with dihydroxyacetone in liquid ammonia under pressure to give 2-R²-5-hydroxymethylimidazole. This intermediate is reacted with acetic anhydride to give 1-acetyl-5-acetoxymethyl-2-R²-imidazole. The diacetate intermediate is N-alkylated, for example, using 2-chlorobenzyl triflate and the resulting 1-R¹(CH₂)ₘ-2-R²-5-acetoxy-methylimidazole is treated with aqueous base, such as 10% sodium hydroxide solution, to give the 1-R¹(CH₂)ₘ-2-R²-5-hydroxymethyl-imidazole intermediate.

Alternatively, the 2-R²S-imidazole compounds are prepared by the following procedure. Benzylamines, substituted by one to three substituents selected from halo, C₁₋₆alkyl, C₁₋₆alkoxy, CN, NO₂, CF₃, CO₂C₁₋₆alkyl, SC₁₋₆alkyl, or SO₂C₁₋ ₆alkyl, are alkylated with a C₁₋₆alkyl chloroacetate, for example methyl chloroacetate, in the presence of a base, such as triethylamine, in a suitable solvent, such as dimethylformamide. The resulting alkylaminoalkyl ester compounds are N-formulated with formic acid in the presence of a suitable solvent, such as xylenes, followed by C-formulation of the carbon alpha to both the amino and the ester groups. Reaction of this intermediate with acidic thiocyanate, preferably potassium thiocyanate, in an inert organic solvent, such as a C₁₋₄alkyl alcohol, produces 1-R¹-(CH₂)ₘ-2-mercapto-5-alkanoate ester imidazole compounds. The free thio group of the ester imidazole is reacted with a halo-R² compound, wherein R² is C₂₋₈alkyl, preferably propyl bromide, in the presence of a suitable base, such as sodium carbonate, in an appropriate solvent, such as ethyl acetate. The ester is reduced to the hydroxymethyl-imidazole intermediate by reduction with a suitable reagent, preferably diisobutyl aluminum hydride, in an appropriate solvent, such as tetrahydrofuran, at a temperature of -78°C to 25°C, preferably at less than -10°C.

The hydroxymethyl group of the hereinbefore prepared intermediate is oxidized to an aldehyde by treatment with a suitable reagent, such as anhydrous chromic acid-silica gel in tetrahydrofuran or, preferably, with activated manganese dioxide, in a suitable solvent, such as benzene or toluene, or preferably methylene chloride, at a temperature of 25°C to 140°C, preferably at 25°C. The 1-R¹(CH₂)ₘ-2-R²X-imidazol-5-carboxaldehydes are reacted with an appropriate phosphonate. The phosphonates are prepared, for example, from trialkyl phosphonoacetates by alkylation with an appropriate halide, mesylate or acetate in the presence of a suitable base, such as sodium hydride, in a suitable solvent, preferably glyme at a reaction temperature of 25°C to 110°C, preferably at 55°C, to provide, for example, the phosphonates. The reaction of the imidazol-5-carboxaldehydes with the phosphonates is performed in the presence of a suitable base, such as a metal alkoxide, lithium hydride or preferably sodium hydride, in a suitable solvent, such as ethanol, methanol, ether, dioxane, tetrahydrofuran, or preferably glyme, at a reaction temperature of 10°C to 50°C, preferably at 25°C, to provide a variable mixture of trans and cis, e.g., (E) and (Z), 1-R¹(CH₂)ₘ-2-R²X-5-CH=C(R⁵)-(COOalkyl)-imidazoles. These isomers are readily separated by chromatography over silica gel in suitable solvent systems, preferably hexane in ethyl acetate mixtures. The esters are hydrolyzed to the acids, 1-R¹-(CH₂)ₘ-2-R²X-5-CH=C(R⁵)COOH-imidazoles, using bases, such as potassium hydroxide, lithium hydroxide or sodium hydroxide, in a suitable solvent system, such as, for example, aqueous alcohols or diglyme. The trans and cis structures of the acids are readily determined by NMR by the NOE protocol, as well as by the biological activities since, generally, the trans (E) isomeric acids are the more potent isomers.

Alternatively, the 1-R¹(CH₂)ₘ-2-R²X-imidazol-5-carboxaldehydes are prepared by the following procedure. Starting 2-R²X-imidazol-5-carboxaldehydes are reacted with an N-alkylating protecting reagent, such as chloromethyl pivalate (POM-Cl), in the presence of a base, such as potassium carbonate, in a suitable solvent, such as dimethylformamide, at a temperature of 20°C to 50°C, preferably at 25°C, to give N-alkylation (e.g., POM-derivation) on the least hindered nitrogen atom of the imidazole nucleus. The 1-R¹(CH₂)ₘ-group is incorporated onto the imidazole by N-alkylation of the above prepared aldehyde with a halomethylbenzene compounds, such as methyl 4-bromomethyl-3-chlorobenzoate, at a temperature of 80°C to 125°C, preferably at 100°C. The protecting group on the 3-nitrogen of the imidazole ring is removed by base hydrolysis, for example using a biphasic mixture of ethyl acetate and aqueous sodium carbonate, to give 1-R¹(CH₂)ₘ-2-R²X-imidazole-5-carboxaldehyde compounds. The Formula (I) compounds can be prepared from these 5-carboxaldehyde compounds by the methods described above.

Compounds of Formula (I), wherein R⁶ is COOH or CO₂C₁-C₂alkyl, and R¹, R², R³, R⁴ and R⁵ are as described in Formula (I), are also prepared by the following procedure.

The 2-R²X-imidazole starting materials are reacted with trimethylsilylethoxymethyl(SEM) chloride to give 1-(trimethylsilyl)ethoxymethyl-2-R²X-imidazole. The reaction is carried out, for example, in the presence of sodium hydride in a solvent such as dimethylformamide. The 5-tributyltin derivatives are prepared by lithiation with, for example, butyllithium in a suitable solvent, preferably diethyl ether, followed by treatment of the lithio imidazole derivative with a tributyltin halide, preferably tri-N-butyltin chloride, at -10°C to 35°C, preferably at 25°C. The 1-SEM-2-R2X-5-tributyltinimidazole is coupled with an a,β-unsaturated acid ester having a leaving group on the β-position, such as a halide or trifluoromethanesulfonyloxy group, for example, BrCR⁴=C(R⁵)(COOalkyl), in the presence of a phosphine ligand, such as bis(diphenyl-phosphino)propane, or triphenylphosphine and a palladium (II) compound, or preferably tetrakis(triphenylphosphine)palladium (0), with or without a base, such as tributylamine, at a temperature of 50°C to 150°C, preferably at 120°C. Both the (E) and (Z) olefinic isomers are prepared by this procedure, and the isomeric esters are readily separated by chromatography over silica gel. The 1-SEM group from the (E) and (Z) isomers is hydrolyzed with acid, for example, aqueous hydrochloric, in a suitable alcoholic solvent, such as methanol or ethanol, and the 1-unsubstituted imidazole derivatives are converted to the 1-t-butoxycarbonyl (t-BOC) imidazoles with di-t-butyl dicarbonate (Hoppe-Seyler's Z. Physiol. Chem., (1976), 357, 1651). The t-BOC esters are alkylated and hydrolyzed with, for example, 2-chlorobenzyl-O-triflate in the presence of a suitable base, preferably diisopropylethylamine, in a suitable solvent, preferably methylene chloride, to afford the 1-(2-chlorophenyl)methyl-imidazole derivatives (esters). The (E) and (Z) isomers are hydrolyzed to the (E) and (Z) acids by the method described above.

Compounds of Formula (I) are also prepared by the following procedure. The 1-R¹(CH₂)ₘ-2-R²X-imidazole-5-carboxaldehydes, prepared as described above, are reacted with a substituted half-acid, half-ester derivative of a malonate, such as ethyl 2-carboxy-3-(2-thienyl)propionate, in the presence of a base, such as piperidine, in a suitable solvent, such as toluene, at a temperature of 80°C to 110°C, preferably at 100°C. The resulting 1-R¹(CH₂)ₘ-2-R²X-5-CH=C(R⁵)COOalkylimidazoles are hydrolyzed to the corresponding Formula (I) acid compounds by alkaline hydrolysis as described above.

Compounds of Formula (I) in which R¹ is 2-chlorophenyl or 4-carboxyphenyl, R² is n-butyl or n-propyl, X is a single bond or S, R³ is H, Cl, or CF₃, R⁴ is methyl, R⁵ is as described in Formula (I), R⁶ is COOH or CO₂C₁-C₂alkyl and other parameters are as described above are prepared as follows. The 1-R¹(CH₂)ₘ-2-R²X-imidazol-5-carboxaldehydes, prepared as described above, are converted to the corresponding alcohols with an organo-metallic derivative or Grignard reagent, preferably methyl lithium, in a suitable solvent, such as tetrahydrofuran. The alcohol is oxidized, for example, using manganese dioxide to give the ketone. The olefinic esters are prepared from the ketone by reaction with appropriate phosphonates to give the (E) and/or (Z) isomers which are readily separated. The acids are prepared from the esters by alkaline hydrolysis as described above.

Compounds of Formula (I) in which R³ is H, Cl or CF₃ are prepared as follows. The 1-R¹-(CH₂)ₘ-2-R²X-imidazol-5-carboxaldehydes are treated with the lithium derivative of a substituted ethyl or methyl ester. These lithio derivatives are prepared from the reaction of lithium diisopropylamide in a suitable solvent, preferably tetrahydrofuran, with an acid ester, such as ROOC-CH₂-(2-thienyl), to generate the a-lithio derivatives at -78°C to -10°C, preferably at -78°C, which are then treated with the imidazol-carboxaldehyde. The intermediate β-hydroxy group of the imidazole ester is converted to a mesylate or an acetate and the mesylate, or preferably the acetate, is heated in a suitable solvent, such as toluene, with one to two equivalents of 1,8-diazo-bicyclo[5.4.0]undec-7-ene, at 50 to 110°C, preferably at 80°C, to afford ester compounds of Formula (I) such as 3-(imidazol-5-yl)-2-(2-thienyl)methyl-2-propenoic acid esters. The (E) isomer is the predominate olefinic isomer. The acids are prepared from the esters by the method described above.

Compounds of Formula (I), wherein R¹ is 2-chlorophenyl or 4-carboxyphenyl, R² is n-butyl or n-propyl, X is a single bond or S, R³ is H, Cl or CF_{3,} R⁴ is H, R⁵ is heterocyclic or a substituted heterocyclic group as described in Formula (I) and R⁶ is COOH, may be prepared by heating 1-R¹-(CH₂)ₘ-2-R²X-imidazol-5-carboxaldehydes at 50°C to 180°C, preferably at 140°C, with an appropriate substituted heterocyclic acetic acid and with acetic anhydride and potassium carbonate to provide unsaturated acids of Formula (I), such as 3-[2-n-butyl-1-(2-chlorophenyl)methyl-1H-imidazol-5-yl]-2-R⁵-2-propenoic acid. The trans olefinic acid is the principal product.

Compounds of Formula (I) in which the R¹ substituent is substituted by hydroxy are formed from Formula (I) compounds in which the R¹ group is substituted by C₁-C₄alkoxy using an ether-cleaving reagent, such as boron tribromide or hydrobromic acid.

Compounds of Formula (I) in which the R¹ substituent is substituted by carboxy are formed from Formula (I) compounds in which the R¹ group is substituted by carbo C₁₋₄alkoxy using basic hydrolysis, such as aqueous sodium or potassium hydroxide in methanol or ethanol, or using acidic hydrolysis, such as aqueous hydrochloric acid.

Compounds of Formula (I) in which the R¹ substituent is substituted by a tetrazol-5-yl group are prepared from the correponding carboxy compounds. For example, Formula (I) acid compounds are reacted with a halogenating agent, such as thionyl chloride, in a suitable solvent, for example benzene, to give the corresponding acid halide compounds. The acid halides are then converted to primary amide compounds in a reaction with concentrated ammonia. Subsequent dehydration of the amides with oxalyl chloride/dimethylformamide in acetonitrile/dimethylformamide yields the nitrile compounds, which are the immediate precursors to the Formula (I) tetrazole compounds. Tetrazole formation is accomplished by reacting the nitriles with azide, preferably aluminum azide prepared in situ by the reaction of sodium azide with aluminum chloride, in a suitable solvent, for example tetrahydrofuran. The Formula (I) compounds in which R⁶ is -CO₂H are prepared from these Formula (I) tetrazole ester compounds by basic hydrolysis as described above.

Pharmaceutically acceptable acid addition salts of compounds of Formula (I) are formed with appropriate organic or inorganic acids by methods known in the art. For example, the base is reacted with a suitable inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Representative examples of suitable acids are maleic, fumaric, benzoic, ascorbic, pamoic, succinic, bismethylenesalicylic, methanesulfonic, ethanedisulfonic, acetic, propionic, tartaric, salicylic, citric, gluconic, aspartic, stearic, palmitic, itaconic, glycolic, p-aminobenzoic, glutamic, benzenesulfonic, hydrochloric, hydrobromic, sulfuric, cyclohexylsulfamic, phosphoric and nitric acids.

Pharmaceutically acceptable base addition salts of compounds of Formula (I) in which R⁶ is COOH are prepared by known methods from organic and inorganic bases, including nontoxic alkali metal and alkaline earth bases, for example, calcium, lithium, sodium, and potassium hydroxide; ammonium hydroxide, and nontoxic organic bases, such as triethylamine, butylamine, piperazine, meglumine, choline, diethanolamine, and tromethamine.

Angiotensin II antagonist activity of the compounds of Formula (I) is assessed by in vitro and in vivo methods. In vitro antagonist activity is determined by the ability of the compounds to compete with ¹²⁵I-angiotensin II for binding to vascular angiotensin II receptors and by their ability to antagonize the contractile response to angiotensin II in the isolated rabbit aorta. In vivo activity is evaluated by the efficacy of the compounds to inhibit the pressor response to exogenous angiotensin II in conscious rats and to lower blood pressure in a rat model of renin dependent hypertension.

### Binding

The radioligand binding assay is a modification of a method previously described in detail (Gunther et al., Circ. Res. 47:278, 1980). A particular fraction from rat mesenteric arteries is incubated in Tris buffer with 80 pM of ¹²⁵I-angiotensin II with or without angiotensin II antagonists for 1 hour at 25°C. The incubation is terminated by rapid filtration and receptor bound ¹²⁵I-angiotensin II trapped on the filter is quantitated with a gamma counter. The potency of angiotensin II antagonists is expressed as the IC₅₀ which is the concentration of antagonist needed to displace 50% of the total specifically bound angiotensin II. The IC₅₀ of (E)-3-[2-n-butyl-1-{4-carboxyphenyl}-1H-imidazo-5-yl]-2-(2-thienyl)methyl-2-propenoic acid is about 250.0 nM.

### Aorta

The ability of the compounds to antagonize angiotensin II induced vasoconstriction is examined in the rabbit aorta. Ring segments are cut from the rabbit thoracic aorta and suspended in organ baths containing physiological salt solution. The ring segments are mounted over metal supports and attached to force displacement transducers which are connected to a recorder. Cumulative concentration response curves to angiotensin II are performed in the absence of antagonist or following a 30-minute incubation with antagonist. Antagonist disassociation constants (K_{B}) are calculated by the dose ratio method using the mean effective concentrations. The K_{B} of (E)-3-[2-n-butyl-1-{4-carboxyphenyl}-1H-imidazo-5-yl]-2-(2-thienyl)methyl-2-propenoic acid is about 25 nM.

### Inhibition of pressor response to angiotensin II in conscious rats

Rats are prepared with indwelling femoral arterial and venous catheters and a stomach tube (Gellai et al., Kidney Int. 15:419, 1979). Two to three days following surgery the rats are placed in a restrainer and blood pressure is continuously monitored from the arterial catheter with a pressure transducer and recorded on a polygraph. The change in mean arterial pressure in response to intravenous injections of 250 mg/kg angiotensin II is compared at various time points prior to and following the administration of the compounds intravenously or orally at doses of 0.1 to 300 mg/kg. The dose of compound needed to produce 50% inhibition of the control response to angiotensin II (IC₅₀) is used to estimate the potency of the compounds.

### Antihypertensive activity

The antihypertensive activity of the compounds is measured by their ability to reduce mean arterial pressure in conscious rats made renin-dependent hypertensive by ligation of the left renal artery (Cangiano et al., J. Pharmacol. Exp. Ther. 208:310, 1979). Renal artery ligated rats are prepared with indwelling catheters as described above. Seven to eight days following renal artery ligation, the time at which plasma renin levels are highest, the conscious rats are placed in restrainers and mean arterial pressure is continuously recorded prior to and following the administration of the compounds intravenously or orally. The dose of compound needed to reduce mean arterial pressure by 30 mm Hg (IC₃₀) is used as an estimate of potency.

The intraocular pressure lowering effects employed in this invention may be measured by the procedure described by Watkins, et al., J. Ocular Pharmacol., 1 (2):161-168 (1985).

The compounds of Formula (I) are incorporated into convenient dosage forms, such as injectable preparations, or for orally active compounds, capsules or tablets. Solid or liquid pharmaceutical carriers are employed. Solid carriers include starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid, such as an ampoule, or an aqueous or nonaqueous liquid suspension.

For topical ophthalmolgic administration, the pharmaceutical compositions adapted include solutions, suspensions, ointments, and solid inserts. Typical pharmaceutically acceptable carriers are, for example, water, mixtures of water and water-miscible solvents such as lower alkanols or vegetable oils, and water soluble ophthalmologically acceptable non-toxic polymers, for example, cellulose derivatives such as methyl cellulose. The pharmaceutical preparation may also contain non-toxic auxiliary substances such as emulsifying, preserving, wetting, and bodying agents, as for example, polyethylene glycols; antibacterial components, such as quarternary ammonium compounds; buffering ingredients, such as alkali metal chloride; antioxidants, such as sodium metabisulfite; and other conventional ingredients, such as sorbitan monolaurate.

Additionally, suitable ophthalmic vehicles may be used as carrier media for the present purpose including conventional phosphate buffer vehicle systems.

The pharmaceutical preparation may also be in the form of a solid insert. For example, one may use a solid water soluble polymer as the carrier for the medicament. Solid water insoluble inserts, such as those prepared from ethylene vinyl acetate copolymer, may also be utilized.

The pharmaceutical preparations are made following conventional techniques of a pharmaceutical chemist involving mixing, granulating, and compressing, when necessary, for tablet forms, or mixing, filling and dissolving the ingredients, as appropriate, to give the desired oral, parenteral, or topical products.

Doses of the compounds of Formula (I) in a pharmaceutical dosage unit as described above will be an efficacious, nontoxic quantity selected from the range of 0.01 - 200 mg/kg of active compound, preferably 1 - 100 mg/kg. The selected dose is administered to a human patient in need of angiotensin II receptor antagonism from 1-6 times daily, orally, rectally, topically, by injection, or continuously by infusion. Oral dosage units for human administration preferably contain from 1 to 500 mg of active compound. Preferably, lower dosages are used for parenteral administration. Oral administration, at higher dosages, however, also can be used when safe and convenient for the patient. Topical formulations contain the active compound in an amount selected from 0.0001 to 0.1 (w/v%), preferably from 0.0001 to 0.01. As a topical dosage unit form, an amount of active compound from between 50 ng to 0.05 mg, preferably 50 ng to 5 mg, is applied to the human eye.

The method of this invention of antagonizing angiotensin II receptors in mammals, including humans, comprises administering to a subject in need of such antagonism an effective amount of a compound of Formula (I). The method of this invention of producing antihypertensive activity and the method of treating congeseive heart failure, glaucoma, and renal failure comprise administering a compound of Formula (I) to a subject in need thereof an effective amount to produce said activity.

The following examples illustrate preparation of compounds and pharmaceutical compositions of this invention. The examples are not intended to limit the scope of this invention as defined hereinabove and as claimed below.

### Example 1

### (E)-3-[2-n-Butyl-1-{4-carboxyphenyl}-1H-imidazo-5-yl]-2-(2-thienyl)methyl-2-propenoic acid

Imidazole was converted to the 1-diethoxyorthoamide derivative by the method of Curtis and Brown, J. Org. Chem., (1980), 45, 20. Imidazole (12.8 g, 0.19 mol) and 118.4 g (0.8 mol) of triethylorthoformate were reacted in the presence of 1 g of p-toluenesulfonic acid to give 20.6 (61%), bp 65-70°C (0.1 mm) of 1-diethoxyorthoamide imidazole. This product (24.0 g, 0.14 mol) was dissolved in dry tetrahydrofuran (250 mL), cooled to -40°C and n-butyl lithium (0.14 mol, 56.4 mL of 2.5 M in hexane) was added at -40°C to -35°C. After 15 minutes n-butyl iodide (31.1 g, 0.169 mol) was added at -40°C, and the reaction was stirred overnight at ambient temperature. The reaction was partitioned between ether and 0.3 N hydrochloric acid, and the organic layer was repeatedly extracted with dilute hydrochloric acid. The combined aqueous extracts were neutralized with sodium bicarbonate solution, extracted with methylene chloride, dried over magnesium sulfate and concentrated. A flash distillation on a Kugelrohr apparatus provided 14.8 g (85%) of 2-n-butylimidazole.

A mixture of 2-n-butylimidazole (12.42 g, 0.10 mol), ethyl 4-fluorobenzoate (19.0 g, 0.113 mol) and potassium carbonate (27.64 g, 0.20 mol) in 100 mL of dimethylsulfoxide was stirred at 135°C under argon for 16 hours. The mixture was cooled, diluted with diethyl ether and filtered. The filter cake was washed thoroughly with diethyl ether. The combined organic extracts were washed with water and brine and then dried with sodium sulfate. The solvent was removed in vacuo to give 16.10 g of crude product. The product was purified by flash chromatography on silica gel eluting with hexane/ethyl acetate (80/20 to 60/40) to give 7.82 g (29%) of 2-n-butyl-1-(4-carboethoxyphenyl)-1H-imidazole.

A mixture of 2-n-butyl-1-(4-carboethoxyphenyl)-1H-imidazole (3.39 g, 0.0124 mol), 37% formaldehyde (11.5 mL, 0.153 mol), sodium acetate (1.82 g, 0.0222 mol) and acetic acid (1.36 mL) was heated to reflux for 40 hours under argon. The reaction was concentrated in vacuo, and the residue was stirred with 500 mL of 20% sodium hydroxide solution for 4 hours, diluted with water and extracted with methylene chloride. The extract was washed, dried, and concentrated. The crude product was flash chromatographed over silica gel with a gradient of ethyl acetate to 10% of methanol in ethyl acetate to give 0.81 g of 2-n-butyl-1-(4-carboethoxyphenyl)-5-hydroxymethyl-1H-imidazole.

To a solution of 2-n-butyl-1-(4-carboethoxyphenyl)-5-hydroxymethyl-1H-imidazole (0.78 g, 0.0026 mol) in methylene chloride (40 mL) was added activated manganese dioxide (2.4 g). The suspension was stirred at room temperature for 18 hours. The solids were filtered and the filtrate concentrated 0.70 g (91%) of 2-n-butyl-1-(4-carboethoxyphenyl)-1H-imidazol-5-carboxaldehyde, as an oil.

Ethyl 2-carboxy-3-(2-thienyl)propionate (14 g, 0.061 mol) was prepared by stirring a solution of diethyl 2-thienylmalonate (16.8 g, 0.0655 mol) and potassium hydroxide (4.41 g, 0.0786 mol) in 200 ml of ethanol under argon at room temperature for 12 days and then purifying by removing the solvent under vacuum, dissolving the residue in water, washing the aqueous layer with aqueous hydrochloric acid and with diethyl ether.

A mixture of the half-acid, half-ester, prepared above, (4.64 g, 0.0203 mol), 2-n-butyl-1-(4-carboethoxyphenyl)-1H-imidazol-5-carboxaldehyde (0.66 g, 0.0022 mol), piperidine (0.56 g, 65 mL, 0.0066 mol), and 3.07 mL of pyridine in 75 mL of benzene was refluxed using a Dean-Stark trap to remove the water. The mixture was refluxed for 2.5 hours. The mixture was concentrated in vacuo, removing traces of pyridine by azeotroping with toluene. The crude product was flash chromatographed on silica gel eluting with hexane/ethyl acetate and then this product was rechromotographed eluting with chloroform/acetone (97.5/2.5) to give 0.79 g of ethyl (E)-3-[2-n-butyl-1-(4-carboethoxyphenyl)-1H-imidazol-5-yl]-2-(2-thienyl)methyl-2-propenoate.

A mixture of ethyl (E)-3-[2-n-butyl-1-(4-carboethoxyphenyl)-1H-imidazol-5-yl]-2-(2-thienyl)methyl-2-propenoate and sodium hydroxide (0.40 g, 0.01 mol) in 2:1 ethanol/water (15 mL) was stirred at room temperature for 18 hours. The mixture was filtered, diluted with 15 mL of water, chilled in an ice bath and then the pH was adjusted to 4.0 with 10% aqueous hydrochloric acid solution. The resulting solid was collected, washed with water and dried to give 0.633 g (95%) of the title compound; mp 260-256°C(d).

### Example 2

### (E)-3-[2-n-Butyl-1-{(4-carboxyphenyl)ethyl}-1H-imidazo-5-yl]-2-(2-thienyl)methyl-2-propenoic acid

The title compound was prepared following the procedure of Example 1; mp 256-259°C(d).

### Example 3

An oral dosage form for administering orally active Formula (I) compounds is produced by screening, mixing and filling into hard gelatin capsules the ingredients in proportions, for example, as shown below.

| Ingredients | Amounts |
|---|---|
| (E)-3-[2-n-butyl-1-{(4-carboxyphenyl)-ethyl}-1H-imidazol-5-yl]-2-(2-thienyl)methyl-2-propenoic acid | 100 mg |
| magnesium stearate | 10 mg |
| lactose | 100 mg |

### Example 4

The sucrose calcium sulfate dihydrate and orally active Formula (I) compounds are mixed and granulated with a 10% gelatin solution. The wet granules are screened, dried, mixed with the starch, talc and stearic acid, screened and compressed into a tablet.

| Ingredients | Amounts |
|---|---|
| (E)-3-[2-n-butyl-1-{4-carboxyphenyl}-1H-imidazol-5-yl]-2-(2-thienyl)methyl-2-propenoic acid | 75 mg |
| calcium sulfate dihydrate | 100 mg |
| sucrose | 15 mg |
| starch | 8 mg |
| talc | 4 mg |
| stearic acid | 2 mg |

### Example 5

(E)-3-[2-n-butyl-1-{(4-carboxyphenyl)ethyl}-1H-imidazol-5-yl]-2-(2-thienyl)methyl-2-propenoic acid, 50 mg, is dispersed in 25 ml of normal saline to prepare an injectable preparation.

### Example 6

A topical ophthalmological solution for administering Formula (I) compounds is produced by mixing under sterile conditions the ingredients in proportions, for example, as shown below.

| Ingredients | Amounts (mg/mL) |
|---|---|
| (E)-3-[2-n-butyl-1-{4-carboxyphenyl}-1H-imidazol-5-yl]-2-(2-thienyl)methyl-2-propenoic acid | 1.0 |
| dibasic sodium phosphate | 10.4 |
| monobasic sodium phosphate | 2.4 |
| chlorobutanol | 5.0 |
| hydroxypropanol methylcellulose | 5.0 |
| sterile water | q.s.ad 1.0mL |
| 1.0 N sodium hydroxide | q.s.ad pH 7.4 |

It is to be understood that the invention is not limited to the embodiments illustrated hereabove and the right to the illustrated embodiments and all modifications coming within the scope of the following claims is reserved.

## Claims

1. A compound of the formula (I): 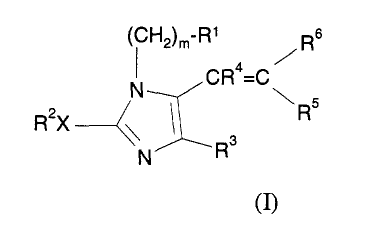 in which:
R¹ is phenyl unsubstituted or substituted by one to three substituents selected from chloro, fluoro, trifluoromethyl, nitro, methyl, methoxy, hydroxy, sulfonamido, cyano, carboxy, carboC₁-C₆alkoxy, carbamoyl, and tetrazol-5-yl;
m is 0 or 2;
X is a single bond or S;
R² is C₂-C₈alkyl;
R³ is hydrogen, chloro, fluoro, or trifluoromethyl;
R⁴ is hydrogen or C₁-C₄alkyl;
R⁵ is thienylmethyl, furylmethyl, imidazolylmethyl, or pyridylmethyl, each of which is unsubstituted or substituted by methyl or methoxy; or R⁵ is benzyl unsubstituted or substituted by one to three substituents selected from C1, Br, F, NO₂, OCH₃, OH, CF₃, NR⁷R⁷, CH₃, or CO₂R⁷, or by methylenedioxy;
R⁶ is CO₂H, CO₂C₁-C₂alkyl, or CONH₂; and
each R⁷ independently is hydrogen, C₁-C₆alkyl, or (CH₂)_{q} phenyl, wherein q is 0-4;
or a pharmaceutically acceptable salt thereof.

2. A compound according to claim 1 which is the E isomer, wherein the R⁶ group and the imidazole are trans to each other.

3. A compound according to claim 1 which is:
(E)-3-[2-n-butyl-1-{4-carboxyphenyl}-1H-imidazol-5-yl]-2-(2-thienyl)methyl-2-propenoic acid; or
(E)-3-[2-n-butyl-1-{(4-carboxyphenyl)ethyl}-1H-imidazol-5-yl]-2-(2-thienyl)methyl-2-propenoic acid;
or a pharmaceutically acceptable salt thereof.

4. A compound according to any one of claims 1 to 3 for use as a medicament.

5. A pharmaceutical composition which comprises a compound according to any one of claims 1 to 3 and a pharmaceutically acceptable carrier.

6. A process for preparing a compound of the formula (I) according to claim 1 in which:
(A) R¹ is 2-chlorophenyl or 4-carboxyphenyl, R² is n-butyl or n-propyl, X is a single bond or S, R³ is hydrogen, chloro, or CF₃, R⁴ is hydrogen, R⁵ is as defined in claim 1, R⁶ is CO₂H, or CO₂C₁-C₂alkyl; or a pharmaceutically acceptable salt thereof, which process comprises reacting a 1-R¹-(CH₂)ₘ-2-R²-X-imidazol-5-carboxaldehyde in which X, R¹ and R² are as hereinbefore defined, with:
(a) an appropriate phosphonate, in the presence of a base,
(b) with a substituted half-acid, half ester derivative of a malonate, in the presence of a base, and thereafter, and if necessary, hydrolysing the resulting ester under alkaline conditions to the corresponding compound of formula (I); or
(c) reacting a compound of the formula (I) as defined in (A) and in which R⁵ is a heterocyclic or substituted heterocyclic group as defined in claim 1 and R⁶ is CO₂H, with an appropriate heterocyclic acetic acid in acetic anhydride in the presence of potassium carbonate; and thereafter where necessary: for formula (I) compounds in which the R¹ group is substituted by carboxy, hydrolyzing the formula (I) compounds in which the R¹ group is substituted by carbo C₁₋₄ alkoxy under basic or acidic conditions;
(B) R⁶ is CO₂H, or CO₂C₁-C₂alkyl, and R¹, R², R³, R⁴, and R⁵ are as defined in claim 1, which process comprises alkylating a corresponding 1-t-butoxycarbonyl imidazole in the presence of a base and thereafter hydrolysing the product, to provide a 1-alkylated imidazole ester and thereafter, and if necessary, hydrolysing the resulting ester to the corresponding compound of formula (I);
(C) R³ is H, C1, or CF₃, which process comprises reacting a mesylate or an acetate of the corresponding β-hydroxy imidazole ester obtainable by treating a 1-R¹-(CH₂)ₘ-2-R²X-imidazol-5-carboxaldehyde with the lithium derivative of a substituted ethyl or methyl ester ROOC-CH₂-R⁵, with 1,8-diazo-bicyclo[5.4.0]undec-7-ene, to form an ester compound of formula (I);
and, thereafter, optionally forming a pharmaceutically acceptable salt.

7. The use of a compound of the formula (I) or a pharmaceutically acceptable salt thereof as defined in any one of claims 1 to 3 in the manufacture of a medicament for the treatment of diseases in which angiotensin II receptor antagonism is a factor.

8. The use of a compound of the formula (I) or a pharmaceutically acceptable salt thereof as defined in any one of claims 1 to 3 in the manufacture of a medicament for the treatment of hypertension.

9. The use of a compound of the formula (I) or a pharmaceutically acceptable salt thereof as defined in any one of claims 1 to 3 in the manufacture of a medicament for the treatment of congestive heart failure

## Patentansprüche

1. Verbindung der Formel (1): 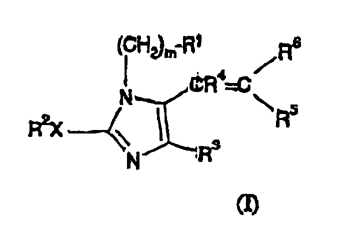 in der:
R¹ eine Phenylgruppe ist, die unsubstituiert oder mit einen bis drei Substituenten, ausgewählt aus Chlor, Fluor, Trifluormethyl, Nitro, Methyl, Methoxy, Hydroxy, Sulfonamido, Cyan, Carboxy, Carbo-C₁-C₆-alkoxy, Carbamoyl und Tetrazol-5-yl, substituiert ist;
m 0 oder 2 ist;
X eine Einfachbindung oder S ist;
R² ein C₂-C₈-Alkylrest ist;
R³ für Wasserstoff, Chlor, Fluor oder Trifluormethyl steht;
R⁴ für Wasserstoff oder einen C₁-C₄-Alkylrest steht;
R⁵ eine Thienylmethyl-, Furylmethyl-, Imidazolymethyl- oder Pyridylmethylgruppe ist, die jeweils unsubstituiert oder mit Methyl oder Methoxy substituiert ist; oder R⁵ eine Benzylgruppe ist, die unsubstituiert oder mit ein bis drei Substituenten, ausgewählt aus Cl, Br, F, NO₂, OCH₃, OH, CF₃, NR⁷R⁷, CH₃ oder CO₂R⁷, oder mit Methylendioxy substituiert ist;
R⁶ für CO₂H, CO₂C₁-C₂-Alkyl oder CONH₂ steht; und jeder Rest R⁷ unabhängig voneinander für Wasserstoff, C₁-C₆-Alkyl oder (CH₂)_{q}-
Phenyl steht, wobei q 0 bis 4 ist;
oder ein pharmazeutisch verträgliches Salz davon.

2. Verbindung nach Anspruch 1, nämlich das E-Isomer, wobei der Rest R⁶ und das Imidazol in der trans-stellung zueinander stehen.

3. Verbindung nach Anspruch 1, nämlich:
(E)-3-[2-n-Butyl-1-{4-carboxyphenyl}-1H-imidazol-5-yl]-2-(2-thienyl)methyl-2-propensäure; oder
(E)-3-[2-n-butyl-1-[(4-carboxyphenyl)ethyl]-1H-imidazol-5-yl]-2-(2-thienyl)methyl-2-propensäure;
oder ein pharmazeutisch verträgliches Salz davon.

4. Verbindung nach einem der Ansprüche 1 bis 3 zur Verwendung als ein Medikament.

5. Arzneimittel, umfassend eine Verbindung nach einem der Ansprüche 1 bis 3 und einen pharmazeutisch verträglichen Träger.

6. Verfahren zur Herstellung einer Verbindung der Formel (I) nach Anspruch 1, wobei:
(A) R¹ für 2-Chlorphenyl oder 4-Carboxyphenyl steht, R² für n-Bulyl oder n-Propyl steht, X eine Einfachbindung oder S ist, R³ für Wasserstoff, Chlor oder CF₃ steht, R⁴ für Wasserstoff steht, R⁵ wie in Anspruch 1 definiert ist, R⁶ für CO₂H oder CO₂C₁-C₂-Alkyl steht; oder ein pharmazeutisch verträgliches Salz davon, wobei das Verfahren umfasst: Umsetzen eines 1-R¹-(CH₂)ₘ-2-R²-X-Imidazol-5-carboxaldehyds, bei dem X, R¹ und R² wie vorstehend definiert sind, mit:
(a) einem geeigneten Phosphonat in Gegenwart einer Base,
(b) mit einem substituierten Halbsäure-Halbester-Derivat eines Malonats in Gegenwart einer Base und danach, und falls notwendig, Hydrolysieren des resultierenden Esters unter alkalischen Bedingungen zur entsprechenden Verbindung der Formel (I); oder
(c) Umsetzen einer Verbindung der Formel (I), die wie in (A) definiert und bei der R⁵ ein heterocyclischer oder substituierter heterocyclischer Rest ist, wie in Anspruch 1 definiert, und R⁶ für CO₂H steht, mit einer geeigneten heterocyclischen Essigsäure in Essigsäureanhydrid in Gegenwart von Kaliumcarbonat und danach, wenn notwendig;
bei Verbindungen der Formel (I), bei denen der Rest R¹ mit Carboxy substituiert ist, Hydrolysieren der Verbindungen der Formel (I), bei denen der Rest R¹ mit Carbo-C₁₋₄-alkoxy substituiert ist, unter basischen oder sauren Bedingungen;
(B) R⁶ für CO₂H oder CO₂C₁-C₂-Alkyl steht und R¹, R², R³, R⁴ und R⁵ wie in Anspruch 1 definiert sind, wobei das Verfahren Alkylieren eines entsprechenden 1-t-Butoxycarbonylimidazols in Gegenwart einer Base und danach Hydrolysieren des Produkts zu einem 1-alkylierten Imidazolester und danach, und falls notwendig, Hydrolysieren des resultierenden Esters zur entsprechenden Verbindung der Formel (1) umfasst;
(C) R³ für H, Cl oder CF₃ steht, wobei das Verfahren Umsetzen eines Mesylats oder Acetats des entsprechenden β-Hydroxyimidazolesters, der durch Behandeln eines 1-R¹(CH₂)ₘ-2-R²X-Imidazol-5-carboxaldehyds mit dem Lithiumderivat eines substituierten Ethyl- oder Methylesters ROOC-CH₂-R⁵ erhältlich ist, mit 1,8-Diazo-bicyclo[5.4.0]undec-7-en zu einer Esterverbindung der Formel (I) umfasst; und danach gegebenenfalls Erzeugen eines pharmazeutisch verträglichen Salzes.

7. Verwendung einer Verbindung der Formel (I) oder eines pharmazeutisch verträglichen Salzes davon, wie in einem der Ansprüche 1 bis 3 definiert, bei der Herstellung eines Medikaments zur Behandlung von Krankheiten, bei denen Angiotensin-IIrezeptorenantagonismus eine Rolle spielt.

8. Verwendung einer Verbindung der Formel (I) oder eines pharmazeutisch verträglichen Salzes davon, wie in einem der Ansprüche 1 bis 3 definiert, bei der Herstellung eines Medikaments zur Behandlung von Hypertonie.

9. Verwendung einer Verbindung der Formel (I) oder eines pharmazeutisch verträglichen Salzes davon, wie in einem der Ansprüche 1 bis 3 definiert, bei der Herstellung eines Medikaments zur Behandlung von Stauungsherzinsuffizienz.

## Revendications

1. Composé de formule (I) : 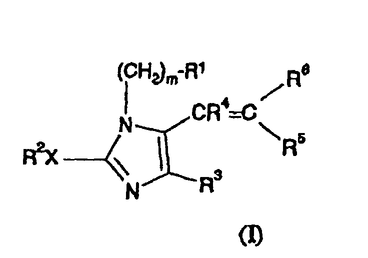 dans laquelle :
R¹ représente un groupe phényle non substitué ou substitué avec un à trois substituants choisis entre des substituants chloro, fluoro, trifluorométhyle, nitro, méthyle, méthoxy, hydroxy, sulfonamido, cyano, carboxy, carbo(alkoxy en C₁ à C₆), carbamoyle et tétrazole-5-yle ;
m est égal à 0 ou 2 ;
X représente une liaison simple ou S ;
R² représente un groupe alkyle en C₂ à C₈ ;
R³ représente l'hydrogène, un groupe chloro, fluoro ou trifluorométhyle ;
R⁴ représente l'hydrogène ou un groupe alkyle en C₁ à C₄ ;
R⁵ représente un groupe thiénylméthyle, furylméthyle, imidazolylméthyle ou pyridylméthyle, chacun de ces groupes étant non substitué ou substitué avec un substituant méthyle ou méthoxy ; ou bien R⁵ représente un groupe benzyle non substitué ou substitué avec un à trois substituants choisis entre des substituants Cl, Br, F, NO₂, OCH₃, OH, CF₃, NR⁷R⁷, CH₃ et CO₂R⁷, ou avec un substituant méthylènedioxy ;
R⁶ représente un groupe CO₂H, CO₂(alkyle en C₁ ou C₂) ou CONH₂ ; et
chaque groupe R⁷ représente, indépendamment, l'hydrogène, un groupe alkyle en C₁ à C₆ ou (CH₂)_{q}phényle, dans lequel q a une valeur de 0 à 4 ;
ou un de ses sels pharmaceutiquement acceptables.

2. Composé suivant la revendication 1, qui est l'isomère E, dans lequel le groupe R⁶ et l'imidazole sont en position trans l'un par rapport à l'autre.

3. Composé suivant la revendication 1, qui est :
l'acide (E)-3-[2-n-butyl-1-{4-carboxyphényl}-1H-imidazole-5-yl]-2-(2-thiényl)méthyl-2-propénoïque; ou
l'acide (E)-3-[2-n-butyl-1-{(4-carboxyphényl)éthyl}-1H-imidazole-5-yl]-2-(2-thiényl)méthyl-2-propénoïque ;
ou un de ses sels pharmaceutiquement acceptables.

4. Composé suivant l'une quelconque des revendications 1 à 3, destiné à être utilisé comme médicament.

5. Composition pharmaceutique qui comprend un composé suivant l'une quelconque des revendications 1 à 3 et un support pharmaceutiquement acceptable.

6. Procédé pour la préparation d'un composé de formule (I) suivant la revendication 1, dans lequel :
(A) R¹ représente un groupe 2-chlorophényle ou 4-carboxyphényle, R² représente un groupe n-butyle ou n-propyle, X représente une liaison simple ou S, R³ représente l'hydrogène, un groupe chloro ou CF₃, R⁴ représente l'hydrogène, R⁵ répond à la définition suivant la revendication 1, R⁶ représente un groupe CO₂H ou CO₂(alkyle en C₁ ou C₂) ; ou d'un de ses sels pharmaceutiquement acceptables, procédé qui comprend la réaction d'un 1-R¹-(CH₂)ₘ-2-R²-X-imidazole-5-carboxaldéhyde dans lequel X, R¹ et R² répondent aux définitions précitées, avec :
(a) un phosphonate approprié, en présence d'une base,
(b) avec un dérivé consistant en hémi-acide/hémi-ester dérivé d'un malonate, en présence d'une base, et ensuite, et si nécessaire, l'hydrolyse de l'ester résultant dans des conditions alcalines en le composé correspondant de formule (I) ; ou
(c) la réaction d'un composé de formule (I) répondant à la définition figurant en (A) et dans laquelle R⁵ représente un groupe hétérocyclique ou hétérocyclique substitué répondant à la définition suivant la revendication 1 et R⁶ représente un groupe CO₂H, avec un acide acétique hétérocyclique approprié dans de l'anhydride acétique en présence de carbonate de potassium ; et ensuite, lorsque cela est nécessaire : pour les composés de formule (I) dans lesquels le groupe R¹ est substitué avec un substituant carboxy, l'hydrolyse des composés de formule (I) dans lesquels le groupe R¹ est substitué avec un groupe carbo(alkoxy en C₁ à C₄) dans des conditions basiques ou acides ;
(B) R⁶ représente un groupe CO₂H ou CO₂(alkyle en C₁ ou C₂), et R¹, R², R³, R⁴ et R⁵ répondent aux définitions suivant la revendication 1, procédé qui comprend l'alkylation d'un 1-tertio-butoxycarbonylimidazole correspondant en présence d'une base, et ensuite l'hydrolyse du produit, ce qui donne un ester d'imidazole 1-alkylé, et ensuite, et si nécessaire, l'hydrolyse de l'ester résultant le composé correspondant de formule (I) ;
(C) R³ représente H, Cl ou un groupe CF₃, procédé qui comprend la réaction d'un mésylate ou d'un acétate de l'ester de β-hydroxyimidazole correspondant pouvant être obtenu en traitant un 1-R¹-(CH₂)ₘ-2-R²X-imidazole-5-carboxaldéhyde avec le dérivé de lithium d'un ester éthylique ou méthylique substitué de formule ROOC-CH₂-R⁵ avec du 1,8-diazo-bicyclo[5.4.0]undéc-7-ène, pour former un ester de formule (I) ;
et ensuite, facultativement, la formation d'un sel pharmaceutiquement acceptable.

7. Utilisation d'un composé de formule (I) ou d'un de ses sels pharmaceutiquement acceptables répondant à la définition suivant l'une quelconque des revendications 1 à 3 dans la production d'un médicament destiné au traitement de maladies dans lesquelles l'antagonisme des récepteurs d'angiotensine II est un facteur.

8. Utilisation d'un composé de formule (I) ou d'un de ses sels pharmaceutiquement acceptables répondant à la définition suivant l'une quelconque des revendications 1 à 3 dans la production d'un médicament destiné au traitement de l'hypertension.

9. Utilisation d'un composé de formule (I) ou d'un de ses sels pharmaceutiquement acceptables répondant à la définition suivant l'une quelconque des revendications 1 à 3 dans la production d'un médicament destiné au traitement de l'insuffisance cardiaque congestive.
